# EUROPÄISCHE PATENTSCHRIFT

(11) **EP 0 660 689 B1**
(45) Veröffentlichungstag und Bekanntmachung des Hinweises auf die Patenterteilung: **27.12.1996**
(21) Anmeldenummer: 93918989.0
(22) Anmeldetag: 11.09.1993
(51) Int. Cl.: A61B 17/60

(54) **OSTEOSYNTHESEHILFSMITTEL**
OSTEOSYNTHESIS AID
AUXILIAIRE D'OSTEOSYNTHESE

(30) Priorität: 19.09.1992 DE 4231443
(43) Veröffentlichungstag der Anmeldung: 05.07.1995
(73) Patentinhaber: Pennig, Dietmar, Dr. med., D-50935 Köln (DE)
(72) Erfinder: Pennig, Dietmar, Dr. med., D-50935 Köln (DE)
(74) Vertreter: Habbel, Hans-Georg, Dipl.-Ing.
(86) Internationale Anmeldenummer: DE9300852
(87) Internationale Veröffentlichungsnummer: WO9406363

(56) Entgegenhaltungen:
- FR-A- 2 301 222
- FR-A- 2 557 933
- FR-A- 2 673 835
- GB-A- 2 101 488
- GB-A- 2 229 096

## Beschreibung

Die Erfindung bezieht sich auf ein Osteosynthesehilfsmittel gemäß dem Oberbegriff des Hauptanspruches.

In der GB-A-22 29 096 wird eine äußere Fixiervorrichtung, insbesondere für Becken beschrieben und dargestellt, die im wesentlichen aus zwei Nagel- oder Schraubenhalteklemmen gebildet ist, wobei diese Nagel- oder Schraubenhalteklemmen unter Zwischenschaltung eines Kugelgelenkes und einer Fixiervorrichtung an ein Mittelteil anschließen. Das Mittelteil besteht dabei aus zwei Anschlußteilen und einem Gelenkkörper, wobei durch den Einsatz von Teleskopführungen zwischen den Anschlußteilen und dem Gelenkkörper eine gewisse Verstellung der Länge des Osteosynthesehilfsmittels möglich ist.

Die bekannte Einrichtung ist recht aufwendig und in ihrer Längeneinstellmöglichkeit eingeschränkt, so daß mehrere Baugrößen bevorratet werden müssen, um dem Operateur die Möglichkeit zu geben, in Anpassung an den jeweiligen Einsatzfall das entsprechende Osteosynthesehilfsmittel einzusetzen.

In der gattungsbildenden GB-A-21 01 488 wird ein äußerer Fixateur beschrieben, bei welchem der Mittelkörper aus wenigstens zwei Bügeln besteht, die je ein Langloch aufweisen, das eine Arretiervorrichtung aufnimmt, die gleichzeitig als Gelenk wirkt. Die Feststellung der beiden Bügel in ihrer Lage zueinander ist von großer Wichtigkeit für die einwandfreie Wirkungsweise des Fixateurs und diese winkelmäßige Arretierung muß auch über einen längeren Zeitraum sicher einstellbar sein. In dieser Literaturstelle werden keine Hilfsmittel erläutert, die eine solche sichere Arretierung der Winkelstellung der beiden Bügel gegeneinander gewährleisten.

Der Erfindung liegt die Aufgabe zugrunde, eine zur Anbringung am Becken geeignete äußere Fixiervorrichtung zu schaffen, die einfach aufgebaut ist, eine große Verstellmöglichkeit aufweist, dreidimensional einstellbar ist, wobei aber die einmal eingestellte Winkelstellung der mehrteiligen Mittelkörper auch über einen langen Zeitraum sicher feststellbar ist.

Diese der Erfindung zugrundeliegende Aufgabe wird durch die Lehre des Hauptanspruches gelöst.

Vorteilhafte Ausgestaltungen sind in den Unteransprüchen erläutert.

Mit anderen Worten ausgedrückt wird vorgeschlagen, daß an die auch beim gattungsbildenden Stand der Technik erforderlichen Fixiereinrichtungen einfache Bügel anschließen, die je ein Langloch aufweisen, das sich vorzugsweise über die ganze Länge des Bügels erstreckt, wobei die Festlegung der beiden Nagel- oder Schraubenhalteklemmen an den Bügeln unter Zwischenschaltung der Fixiereinrichtungen erfolgt. Es ist ersichtlich, daß die Bügel nunmehr auf ein Mindestmaß zusammengeschoben werden können, soweit, daß die Fixiereinrichtungen bereits aneinander anstoßen und daß sie auf ein maximal es Maß auseinandergezogen werden können, soweit, daß eine Arretiervorrichtung im äußeren Bügelscheitel liegt. Die Arretiervorrichtung, um die beiden Bügel in ihrer auseinandergezogenen Stellung und Winkelstellung festzulegen, besteht aus miteinander zusammenwirkenden Bauteilen, die die Bügel umgreifen und unter Zwischenschaltung von Rändelscheiben eine sichere Festlegung der Winkelstellung ermöglichen.

Ein Ausführungsbeispiel der Erfindung wird nachfolgend anhand der Zeichnung erläutert. Die Zeichnung zeigt in
- Fig. 1: eine Ansicht auf eine Ausführungsform der Erfindung und in
- Fig. 2: ein Verbindungsmittel für die beiden Fixatoreinheiten gemäß Fig. 1.

In der Zeichnung ist ein Osteosynthesehilfsmittel dargestellt, das zwei Nagel- oder Schraubenhalteklemmen 2, 3 an sich bekannter Bauart aufweist. An diese Schraubenhalteklemmen 2 und 3 schließen Kugelgelenke 4 und 5 an, an die wiederum Fixiervorrichtungen 6 und 7 anschließen, mit denen ein Mittelkörper 1 gehalten wird.

Auch die Fixiervorrichtungen 6 und 7 sind bekannter Bauart, so daß sie hier nicht weiter erläutert werden müssen.

Der Mittelkörper 1 besteht aus zwei Bügeln 8 und 9, die durch einen schmalen Randsteg oder Rahmen gebildet werden und somit sich über die gesamte Länge erstreckende Langlöcher 10 und 11 aufweisen. Die Langlöcher 10 und 11 werden von einer Arretiervorrichtung 30 durchquert. Diese Arretiervorrichtung 30 dient einerseits zum Arretieren der beiden Bügel 8, 9 in einer vorgegebenen Längenstellung und andererseits dient die Arretiervorrichtung 30 als Gelenk, so daß um die Arretiervorrichtung 30 herum die Bügel 8 und 9 winkelmäßig einstellbar und feststellbar sind.

Es ist erkennbar, daß die Nagel- oder Schraubenhalteklemmen 2 und 3 bzw. die Fixiervorrichtungen 6 und 7 nunmehr so auf ein Maß zusammengeschoben werden können, daß sich die Fixiervorrichtungen 6 und 7 nahezu berühren und daß andererseits diese Bauteile so weit auseinandergezogen werden können, daß die Arretiervorrichtung 30 im Scheitel der Bügel 8 und 9 liegt.

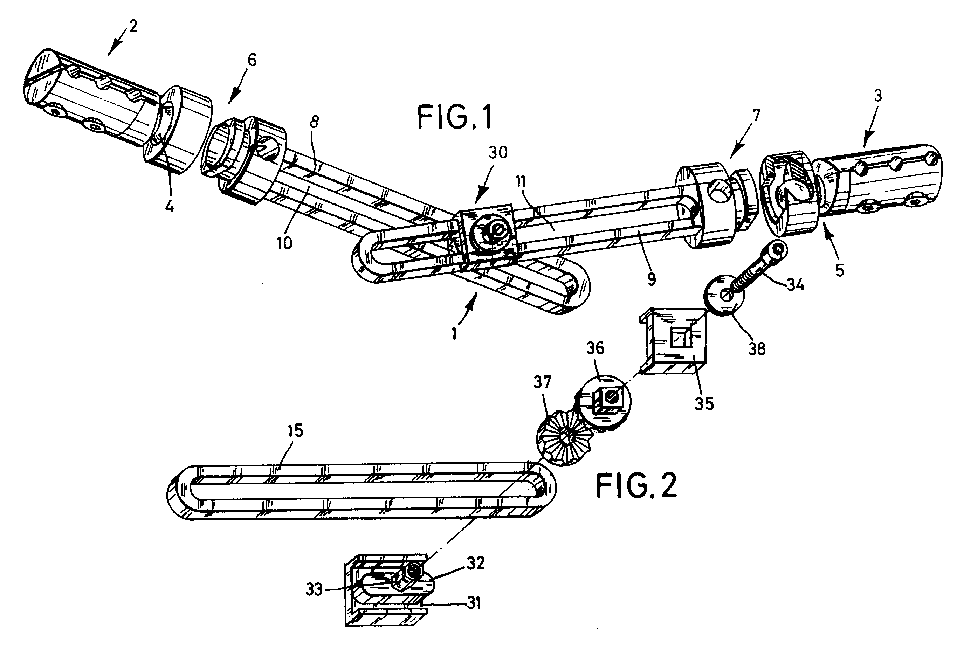

In Fig. 2 ist weiterhin ein Einsatzbügel 15 dargestellt mit einer zusätzlichen Arretiervorrichtung, der nunmehr zwischen die beiden Bügel 8 und 9 eingeschaltet werden kann, so daß auch ein Fixieren des Beckens bei Patienten erfolgen kann, die einen übermäßigen Bauchumfang aufweisen.

Nur der Vollständigkeit halber wird darauf hingewiesen, daß die Festlegung der Nagel - oder Schraubenhalteklemmen 2, 3 vorzugsweise zwischen dem oberen und unteren vorderen Darmbeinstachel oder Beckenkamm erfolgt.

Die Arretiervorrichtung 30 besteht aus einem Schlitten 31 mit einem Mittelstück 32, wobei in den so gebildeten Aufnahmeräumen die Bügelteile aufgenommen werden können. Von dem Mittelstück 32 steht eine mit Innengewinde versehene Hülse 33 vor, in die sich eine Festlegschraube 34 einschrauben kann. Die Festlegschraube 34 durchgreift eine Haube 35, die sich über den anderen der beiden festzulegenden Bügel legt, wobei die Winkelverstellung zwischen den beiden oder den drei Bügeln, gleichzeitig aber auch die Festlegung in der gewünschten Winkelstellung dieser Bügel, durch zwei Rändelscheiben 36 und 37 erfolgt, bei deren Lösen ein Verstellen gegeneinander möglich ist, aber bei deren Aufeinanderpressen eine winkelmäßige Arretierung erfolgt. Hierbei durchgreift die Rändelscheibe 36 mit einem Vierkant eine entsprechende Vierkantöffnung in der Haube 35 und die Festlegschraube 34 legt sich unter Zwischenschaltung einer Unterlegscheibe 38 mit ihrem Kopf auf der Oberseite der Haube 35 fest.

Wie dies die Darstellung in Fig. 1 zeigt, ist es hiermit möglich, eine beliebige Einstellung des Winkels der beiden Bügel 8 und 9 bzw. der drei Bügel 8, 9 und 15 gegeneinander zu erreichen, gleichzeitig aber auch die Winkelstellung fest zu arretieren, so daß die gewünschte Festlegung erfolgt.

## Patentansprüche

1. Osteosynthesehilfsmittel für die Fixation von Knochen, insbesondere des Beckens, mit Nagel- oder Schraubenhalteklemmen (2, 3), die an einen mehrteiligen und längenveränderlichen Mittelkörper (1) anschließen, wobei der Mittelkörper (1) aus zwei Bügeln (8, 9) mit je einem Langloch (10, 11) gebildet ist und die Langlöcher (10, 11) eine Arretiervorrichtung (30) aufnehmen, die als Gelenk dient und die Bügel (8, 9) in ihrer Winkellage und Längeneinstellung zueinander arretieren kann,
dadurch gekennzeichnet, daß
a) die Nagel- oder Schraubenhalteklemmen (2, 3) unter Zwischenschaltung je eines Kugelgelenkes (4, 5) und je einer Fixiereinrichtung (6, 7) an den Mittelkörper anschließen,
b) die Arretiervorrichtung (30) einen Schlitten (31) mit einem Mittelstück (32) und beiderseits des Mittelstückes (32) ausgebildeten Aufnahmeräumen aufweist, wobei sich das Mittelstück (32) in das Langloch (10, 11) einlegt und die Aufnahmeräume den Bügel (8, 9) aufnehmen,
c) von dem Mittelstück (32) eine mit Innengewinde versehene Hülse (33) vorsteht, in die eine Schraube (34) einschraubbar ist, die sich auf einer Haube (35) abstützt, die sich über den anderen der beiden festzulegenden Bügel (8, 9) legt,
d) die Arretiervorrichtung (30) Rändelscheiben (36, 37) aufweist,
e) in der Haube (35) eine Vierkantöffnung vorgesehen ist, in die ein an der dem Schlitten (31) abgewandten Seite der Rändelscheibe (36) vorgesehener Höcker eingreifen kann.

2. Osteosynthesehilfsmittel nach Anspruch 1, gekennzeichnet durch einen zusätzlichen Einsatzbügel (15), der zwischen die Bügel (8, 9) einsetzbar ist und ein zweites Arretiermittel zur Verbindung des Einsatzbügels (15) mit den Bügeln (8, 9) in Verbindung mit dem Arretiermittel (30).

3. Osteosynthesehilfsmittel nach Anspruch 1 oder 2, dadurch gekennzeichnet, daß sich die Langlöcher über die gesamte Länge der Bügel (8, 9, 15) erstrecken.

## Claims

1. An osteosynthetic aid for fixing bones, especially in the pelvis, with nail or screw holding clips (2, 3) which are connected to a multi-part, length-adjustable central member (1), the central member (1) consisting of two shackles (8, 9) each with an elongate hole (10, 11) and the elongate holes (10, 11) accommodating a locking device (30) which acts as a hinge and may lock the shackles (8, 9) with respect to each other in their angular position and longitudinal setting,
characterised in that
a) the nail or screw holding clips (2, 3) are each connected to the central member via a ball-and-socket joint (4, 5) and a fixing device (6, 7),
b) the locking device (30) comprises a slide block (31) with a central piece (32) and receiving spaces formed on both sides of the central piece (32), the central piece (32) being inserted into the elongate hole (10, 11) and the receiving spaces accommodating the shackle (8, 9),
c) an internally threaded sleeve (33) projects from the central piece (32), into which sleeve (33) a screw (34) may be screwed which is supported on a cap (35) which lies over the other one of the two shackles (8, 9) to be fixed,
d) the locking device (30) comprises knurled disks (36, 37),
e) a square opening is provided in the cap (35), in which opening there may engage a projection provided on the side of the knurled disk (36) remote from the slide block (31).

2. An osteosynthetic aid according to claim 1, characterised by an additional insert shackle (15), which may be inserted between the shackles (8, 9), and a second locking means for connecting the insert shackle (15) with the shackles (8, 9) in conjunction with the locking means (30).

3. An osteosynthetic aid according to claim 1 or claim 2, characterised in that the elongate holes extend over the entire length of the shackles (8, 9, 15).

## Revendications

1. Auxiliaire d'ostéosynthèse pour la fixation d'os, en particulier d'os du bassin, avec des pinces de maintien pour clous ou vis (2, 3) qui se raccordent à un corps central (1) en plusieurs parties et de longueur modifiable, le corps central (1) se composant de deux barrettes (8, 9) possédant chacune un trou allongé (10, 11), et les trous allongés (10, 11) recevant un dispositif de blocage (30) qui sert d'articulation et peut bloquer les barrettes (8, 9) dans leur position angulaire et leur longueur de réglage l'une par rapport à l'autre, caractérisé en ce que
a) les pinces de maintien pour clous ou vis (2, 3) se raccordent au corps central avec intercalation d'une articulation à rotule (4, 5) et d'un dispositif de fixation (6, 7) pour chacune,
b) le dispositif de blocage (30) présente un patin (31) pourvu d'une pièce centrale (32) et de logements ménagés des deux côtés de la pièce centrale (32), la pièce centrale (32) entrant dans le trou allongé (10, 11) et les logements recevant la barrette (8, 9),
c) une douille (33) faisant saillie à partir de la pièce centrale (32) est pourvue d'un filet intérieur, dans laquelle peut être introduite une vis (34) qui s'appuie sur un capuchon (35) reposant sur l'autre barrette à fixer (8, 9),
d) le dispositif de blocage (30) présente des rondelles moletées (36, 37),
e) il est prévu dans le capuchon (35) une ouverture carrée dans laquelle peut se mettre en prise une saillie prévue sur la face de la rondelle moletée (36) opposée au patin (31).

2. Auxiliaire d'ostéosynthèse selon la revendication 1, caractérisé en ce qu'il comporte une barrette à insérer (15) supplémentaire, qui peut être insérée entre les barrettes (8, 9), et un deuxième moyen de blocage destiné à relier la barrette à insérer (15) avec les barrettes (8, 9) en liaison avec les moyens de blocage (30).

3. Auxiliaire d'ostéosynthèse selon la revendication 1 ou 2, caractérisé en ce que les trous allongés s'étendent sur toute la longueur des barrettes (8, 9, 15).
